# EUROPÄISCHE PATENTSCHRIFT

(11) **EP 1 663 076 B1**
(45) Veröffentlichungstag und Bekanntmachung des Hinweises auf die Patenterteilung: **09.07.2008**
(21) Anmeldenummer: 04738506.7
(22) Anmeldetag: 12.05.2004
(51) Int. Cl.: A61F 2/36

(54) **STECKADAPTER FÜR EINE HÜFTENDOPROTHESE**
PLUG ADAPTER FOR A HIP ENDOPROSTHESIS
ADAPTATEUR ENFICHABLE CONÇU POUR UNE ENDOPROTHESE DE HANCHE

(30) Priorität: 14.05.2003 DE 10322174
(43) Veröffentlichungstag der Anmeldung: 07.06.2006
(73) Patentinhaber: Merete Medical Gmbh, 12247 Berlin (DE)
(72) Erfinder: ANAPLIOTIS, Emmanuel, 14195 Berlin (DE); KRANZ, Curt, 14163 Berlin (DE)
(74) Vertreter: Hannig, Wolf-Dieter
(86) Internationale Anmeldenummer: PCT/DE2004/000989
(87) Internationale Veröffentlichungsnummer: WO 2004/103211

(56) Entgegenhaltungen:
- EP-A- 0 202 141
- WO-A-00/69372
- FR-A- 2 606 628
- US-A- 4 032 994
- US-A1- 2002 193 882

## Beschreibung

Die Erfindung betrifft einen Steckadapter für eine Hüftendoprothese mit einer besonders ausgebildeten konusförmigen Aufnahme für den Prothesenschaft auf der einen Seite und einem Aussenkonus für die Verbindung mit einer Gelenkkugel auf der anderen Seite, wobei die Steckverbindung eine erhöhte Festigkeit aufweist.

Die Verwendung von Steckadaptern mit konusförmiger Aufnahme für Endoprothesen, als Bindeglied zwischen dem Schaft einer implantierten Hüftendoprothese und der Steckkugel ist allgemein bekannt.

Ein derartiges Steckadapter-System ist beispielsweise in der US-PS 5,362,311 beschrieben worden, bei dem auf einem konusförmigen Schaft einer implantierten Hüftendoprothese ein länglicher Steckadapter mit einem Innen-und Aussenkonus aufgebracht werden kann, wobei auf dem Aussenkonus des Steckadapters eine Gelenkkugel mit entsprechendem Innenkonus aufgesetzt wird. Der konusförmige Hals des Prothesenschaftes geht dabei eine Konus-Steckverbindung über den Steckadapter mit der Steckkugel ein. Ziel dieser Lösung ist ein Steckadapter, der eine Anpassung der Länge des Prothesenschaftes an die Beinlänge des jeweiligen Patienten ermöglicht.

Der in der WO00/69372 beschriebene Steckadapter soll der der Positionierung des Keramikkugelkopfes zur Endoprothese dienen bzw. die Auslenkung der künstlichen Gelenkpfanne gegenüber der Gelenkprothese beeinflussen. Eine im unteren Endbereich des Adapters vorgesehene Umfangsnut dient der Erhöhung der Beweglichkeit des Kopfes. Die Elastizität des Adapters bleibt dabei unverändert, da der Adapter im Bereich seines größten Durchmessers ungeschwächt ist.

Auch bei dem aus der FR2606628A bekannten Steckadapter im unteren Endbereich des Adapters eine umlaufende Nut vorgesehen, deren Form aber nicht zu einer Elastizitätsvergrößerung des Adapters beiträgt.

Um einen festen Sitz der Konusverbindung zu sichern, werden hohe Anforderungen sowohl an die Oberflächenausbildung des Aussenkonus des implantierten Schaftes als auch an den Innenkonus des Steckadapters gestellt. Gleichzeitig darf die Stabilität und Festigkeit der Wandstärke eines Steckadapters nicht beeinträchtigt werden.

Der Erfindung liegt die Aufgabe zugrunde, einen Steckadapter zu schaffen, der über einen verbesserten Festsitz der Konusverbindung bei unveränderter fertigungsbedingter Rauheit der Oberflächen des Innenkonus und des Aussenkonus auch dann verfügt, wenn - wie es im Falle von Revisionsoperationen vorkommt - der Prothesenkonus Schädigungen aufweist.

Erfindungsgemäß wird diese Aufgabe dadurch gelöst, dass der Adapter an der Seite des größten Durchmessers eine Materialaussparung aufweist, durch die der Konus eine Erhöhung der Elastizität erfährt. Durch die grössere Elastizität des Konus werden Konusfehler kompensiert und fertigungsbedingte Rauheiten ausgeglichen. Zwischen dem Innenkonus und dem Aussenkonus entsteht durch den verbesserten Kontakt der Oberflächen eine grössere Festigkeit der Verbindung, Spannungsspitzen werden vermieden. Die Kompensation der Konusfehler ist beim Einsatz von Steckadaptern bei Revisionsoperationen von besonderer Bedeutung, da festsitzende Prothesen, deren Konus beschädigt ist, dann nicht zwingend ausgetauscht werden müssen.

Die U-förmige Aussparung ist annähernd parallel zur Konusinnenwand bzw. zur Mittellinie des Steckadapters ausgeführt. Durch die abnehmende Dicke der Wandung des Steckadapters zu der Seite seines grössten Durchmessers hin erfolgt die elastische Anpassung an den Aussenkonus der Endoprothese.

Durch Aufschieben des Steckadapters wird dieser elastisch erweitert und es kommt zu einer gewollten Verklemmung.

Günstige Werte hinsichtlich der Elastizität wurden erreicht, wenn die Tiefe der Aussparung über 20% der Sitzlänge des Konus beträgt. Die Tiefe der Aussparung kann bis zu 40% der Sitzlänge des Konus betragen.

Im folgenden werden bevorzugte Ausführungsformen der Erfindung anhand der Zeichnungen näher dargestellt. Es zeigen:

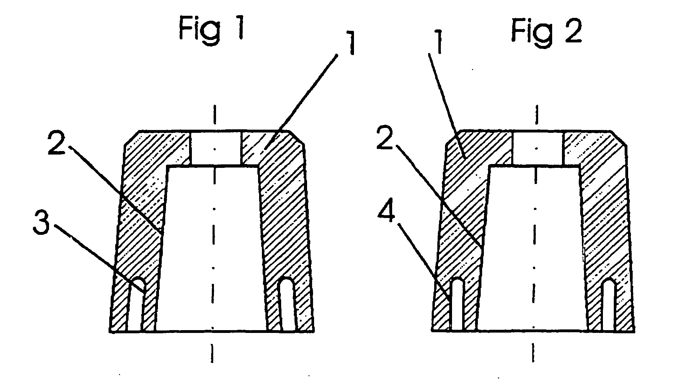

Fig. 1 einen Schnitt durch einen Steckadapter in der erste Ausführungsform mit einer U-förmigen Aussparung parallel zur Konusinnenwand.
Fig. 2 einen Schnitt durch einen Steckadapter in der zweiten Ausführungsforr mit einer U-förmigen Aussparung annähernd parallel zur Mittellinie des Adapters.

### Liste der Bezugszeichen:

- 1: Steckadapter
- 2: Konus
- 3: U-förmige Aussparung, annähernd parallel zur Konusinnenwand
- 4: U-förmige Aussparung, annähernd parallel zur Mittellinie
- 5: langgezogene fasenförmige Aussparung, annähernd parallel zur Mittellinie
- 6: V-förmige Aussparung

## Patentansprüche

1. Steckadapter für eine Hüftendoprothese mit einer konusförmigen Aufnahme für den Prothesenschaft auf der einen Seite und einem Aussenkonus für die Verbindung mit einer Gelenkkugel auf der anderen Seite, wobei die Steckverbindung eine erhöhte Festigkeit aufweist, **dadurch gekennzeichnet, dass** der Adapter an der Seite des grössten Durchmessers eine U- oder V-förmige Materialaussparung aufweist, die sich parallel zur Konusinnenwand oder eine U-förmige Materialaussparung aufweist, die sich parallel zur Mittellinie des Konus erstreckt, so daß der Konus eine Vergrößerung seiner Elastizität erfährt.

2. Steckadapter nach Anspruch 1, **dadurch gekennzeichnet, dass** die Tiefe der Aussparung über 20% und bis zu 40% der Sitzlänge des Konus beträgt.

## Claims

1. A plug adapter for a hip endoprosthesis with a cone shaped seating for the prosthesis shaft on the one side and an external cone for the connection to a joint ball on the other side, wherein the plug connection has an increased strength, **characterized in that** the adapter on the side of the greatest diameter has an U-shaped or V-shaped material recess extending parallel to the internal surface of the cone or a V-shaped material recess extending parallel to the median line of the cone, so as to increase the elasticity of the cone.

2. The plug adapter according to claim 1, **characterized in that** the depth of the recess amounts to more than 20 % and up to 40 % of the seating length of the cone.

## Revendications

1. Adaptateur enfichable pour une endoprothèse de hanche, comprenant un logement conique pour la tige de prothèse sur un côté et un cône extérieur pour la liaison avec une rotule d'articulation sur l'autre côté, dans lequel la liaison enfichable présente une résistance augmentée, **caractérisé en ce que** l'adaptateur présente sur le côté du diamètre le plus grand un évidement de matière en forme de « U » ou en forme de « V » qui s'étend parallèlement à la paroi intérieure du cône ou un évidement de matière en forme de « U » qui s'étend parallèlement à la ligne médiane du cône de telle sorte que le cône se voit augmenté dans son élasticité.

2. Adaptateur enfichable selon la revendication 1, **caractérisé en ce que** la profondeur de l'évidement se monte à plus de 20% et jusqu'à 40% de la longueur d'assise du cône.
